Europäisches Patentamt

European Patent Office

Office européen des brevets

(19)

(11) **EP 1 479 387 A1**

(12) **EUROPEAN PATENT APPLICATION**
published in accordance with Art. 158(3) EPC

(43) Date of publication:
**24.11.2004 Bulletin 2004/48**

(51) Int Cl.[7]: **A61K 31/5575**, A61P 27/06
// A61K31:5575, A61K31:551

(21) Application number: **03734863.8**

(22) Date of filing: **28.01.2003**

(86) International application number:
**PCT/JP2003/000786**

(87) International publication number:
**WO 2003/063879 (07.08.2003 Gazette 2003/32)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **29.01.2002 JP 2002020542**

(71) Applicant: **SANTEN PHARMACEUTICAL CO.,
LTD.
Higashiyodogawa-ku, Osaka-shi,
Osaka 533-8651 (JP)**

(72) Inventors:
• **ICHIKAWA, Masaki,
SANTEN PHARMACEUTICAL CO., LTD.
Ikoma-shi, Nara 630-0101 (JP)**

• **NAKAZAWA, Fumio,
SANTEN PHARMACEUTICAL CO., LTD.
Ikoma-shi, Nara 630-0101 (JP)**
• **HARA, Hideaki,
SANTEN PHARMACEUTICAL CO., LTD.
Ikoma-shi, Nara 630-0101 (JP)**

(74) Representative:
**ter Meer, Nicolaus, Dipl.-Chem., Dr. et al
TER MEER STEINMEISTER & PARTNER GbR,
Patentanwälte,
Mauerkircherstrasse 45
81679 München (DE)**

(54) **REMEDIES FOR GLAUCOMA COMPRISING BUNAZOSIN AND PROSTAGLANDINS**

(57) The object of the present invention is to find utility of a combination of bunazosin, having a new action mechanism, and prostaglandins as a therapeutic agent of glaucoma. The invention relates to a therapeutic agent of glaucoma, comprising bunazosin or a salt thereof and prostaglandins in combination, where bunazosin or a salt thereof and prostaglandins mutually supplement and/or enhance each action, and where bunazosin and prostaglandin may be formulated in a single preparation to be administered or may be separately administered in combination.

EP 1 479 387 A1

**Description**

Technical Field

The present invention relates to a therapeutic agent of glaucoma, the therapeutic agent comprising bunazosin or a salt thereof and prostaglandins in combination.

Background of the Invention

Glaucoma is an intractable ocular disease with a risk of blindness, involving the increase of intraocular pressure due to various factors. Studies have been done about various treating methods therefor. The method for lowering intraocular pressure includes three approaches, namely pharmacotherapy, laser therapy and surgery. For the pharmacotherapy, drugs such as β-blockers, prostaglandin-related drugs, carbonic anhydrase inhibitors, cholinergic agents, and epinephrine-related drugs have been used.

Lately, bunazosin hydrochloride, an α1 blocker, was developed as a drug based on a new action mechanism. Bunazosin hydrochloride is a selective blocker of sympathetic nerve α1 receptor and promotes the uveoscleral outflow to thereby lower intraocular pressure (Folia Ophthalmologica Japonica, 46, 1066-1070, 1995).

As a combination of α1 blocker and another therapeutic agent of glaucoma, Japanese Patent Laid-Open Publication (hereinafter referred to as JP-A) 2001-33551 describes a therapeutic agent of glaucoma comprising an α1 blocker and an angiotensin II antagonist.

Meanwhile, Japanese Patent No. 2726672 states a combination of an adrenergic receptor blocker and prostaglandins, as a therapeutic agent of glaucoma comprising a combination of prostaglandins and another therapeutic agent. However, this Japanese patent has no description about the effect of α blockers because β blockers in this patent are mainly adrenergic blockers. In particular, this Japanese patent does not even suggest the effect of α1 blocker.

As described above, no report has been issued about the effect on treating glaucoma by a combination of an α1 blocker and prostaglandins. In particular, studies about bunazosin, an α1 blocker having a novel action mechanism, in combination with prostaglandins have never been done.

Accordingly, it has been a very interesting subject matter to find utility of a combination of bunazosin having a new action mechanism and prostaglandins as a therapeutic agent of glaucoma.

Disclosure of the Invention

The present inventors made intensive studies about the possibility of the development of a therapeutic agent of glaucoma by combining bunazosin with prostaglandins. Consequently, the inventors found that the combination thereof enhanced the action of lowering intraocular pressure , thereby completing the present invention. The detailed test method and the results thereof are described below in the section of Pharmacological Test. Bunazosin in combination with prostaglandins exhibited a remarkable action of lowering intraocular pressure. Additionally, the therapeutic agent of glaucoma in accordance with the present invention can be used preferably not only for the treatment of glaucoma but also for the prevention thereof.

The present invention relates to a therapeutic agent of glaucoma comprising bunazosin or a salt thereof and prostaglandins in combination. These drugs mutually supplement and/or enhance their actions.

For the treatment of glaucoma, bunazosin and prostaglandins may be formulated in a single preparation to be administered. In other words, these drugs may be administered in mixture. Alternatively, each drug may be in a separate preparation and these drugs may be administered in combination.

Bunazosin can be in the form of its salt. Examples of the salts include salts thereof with inorganic acids such as hydrochloric acid and nitric acid. In particular, the hydrochloride salt is preferable.

Since the present invention is characterized by treating glaucoma with bunazosin and prostaglandins in combination, any prostaglandins having the action of lowering intraocular pressure and utility in treating glaucoma may be used, with no specific limitation. Prostaglandins having the action of lowering intraocular pressure are exemplified by prostaglandins described in JP-A-Sho59-1418 (natural prostaglandins, particularly prostaglandin F2α), prostaglandins such as latanoprost as described in Published Japanese Translation of PCT No. 3-501025, prostaglandins such as isopropyl unoprostone as described in JP-A-Hei2-108, prostaglandins such as bimatoprost as described in Published Japanese Translation of PCT No. 8-501310, and prostaglandins such as travoprost as described in JP-A-Hei10-182465. In particular, latanoprost, isopropyl unoprostone, bimatoprost or travoprost, which has already been on the market as a therapeutic agent of glaucoma, is preferably used. It is needless to say that these prostaglandins may be in salt forms or ester forms thereof.

According to the embodiment of the invention, the formulation can be either one formulation containing bunazosin and prostaglandins in mixture or two separate formulations containing each component. Not any specific technique is needed for the preparation of these formulations. They are prepared by widely used methods. Preferably, these formulations are topically administered to eyes. The dosage forms are exemplified by eye drops and eye ointments.

The separate formulations containing bunazosin and prostaglandins respectively can be prepared according to known methods. They are exemplified by the formulation disclosed in Japanese Patent Publication No. 2610619, the formulation disclosed in Japanese Examined Patent Publication Hei 7-23302, and commercially available formulations. The formulation of prostaglandins can be prepared with reference to the descriptions of the above-mentioned Japanese patent laid-open publications. Commercially available formulations of latanoprost, isopropyl unoprostone and the like as glaucoma-treating agents can be used.

The formulation containing bunazosin and prostaglandins in mixture can be also prepared according to known methods. The eye drops can be prepared, using isotonic agents such as sodium chloride and concentrated glycerin; buffers such as sodium phosphate buffer and sodium acetate buffer; surfactants such as polyoxyethylene sorbitan monooleate, stearate polyoxyl 40, and polyoxyethylene hardened castor oil; stabilizers such as sodium citrate and sodium edetate; and preservatives such as benzalkonium chloride and paraben, as needed. The pH should be within an ophthalmologically acceptable range and is preferably within a range of pH 4 to pH 8. For reference, a formulation example thereof is described below in the section of Example. However, the formulation example never limits the scope of the invention.

The doses of bunazosin and prostaglandins can be determined depending on the symptom and age of patients, the dosage form, the administration route and the like. In case of an administration through eye drops, for example, bunazosin is administered generally within 2 to 40 μg daily from once to several times a day. The dose of prostaglandins varies depending on the prostaglandin type. The dose can be determined on the basis of the actual dose range for treatment and is raised or lowered depending on the symptom of patients and the like. The daily dose is within a range of 1 to 1,000 μg, which is administered from once to several times a day. More specifically, isopropyl unoprostone and latanoprost are generally administered at a daily dose of 30 to 300 μg and a daily dose of 1 to 5 μg, respectively. Depending on the symptom of patients and the like, the doses are varied. Based on similar standards, the doses of other prostaglandins can be determined. These doses are also applicable to the administration of bunazosin and prostaglandins in combination. In case that bunazosin and prostaglandins are to be administrated in one formulation, the formulation should be prepared by selecting the mixing ratio of two drugs appropriately so that their daily doses might not excess each dose of the separate drugs. The mixed formulation is administered from once to several times daily.

A formulation example and a pharmacological test are shown in the following Example. The Example is for better understanding of the invention but never limits the scope of the invention.

Best Mode For Carrying out the Invention

[Formulation Example]

A general formulation example of eye drops containing bunazosin and prostaglandins in mixture in accordance with the invention is described below.

| Eye drops (in 100 mL) | |
|---|---|
| Bunazosin hydrochloride | 0.01 g |
| Latanoprost | 0.005 g |
| Boric acid | 0.5 g |
| Concentrated glycerin | 2.0 g |
| Benzalkonium chloride | 0.01 g |
| Dilute hydrochloric acid | q.s. |
| Distilled water | q.s. |

[Pharmacological Test]

So as to study the utility of bunazosin in combination with prostaglandin, bunazosin and prostaglandin were administered in combination to cynomolgus monkeys (Macaca fascicularis), examining the effect on intraocular pressure. Latanoprost was used as prostaglandin.

(Test compound solution)

For bunazosin administration, Detantol (trade mark) ophthalmic solution, which contains bunazosin hydrochloride at 0.01 %, was used. For latanoprost administration, Xalatan (trade mark) ophthalmic solution, which contains latanoprost at 0.005 %, was used.

(Dosing groups)

Cynomolgus monkeys were divided into four groups, a group to be dosed with a vehicle (vehicle group) , a group to be dosed with bunazosin (bunazosin group), a group to be dosed with latanoprost (latanoprost group) and a group to be dosed with bunazosin and latanoprost [(bunazosin + latanoprost) group].

(Administration method and measurement method)

1. For topical anesthesia, a drop of 0.4 % oxybuprocain hydrochloride ophthalmic solution was instilled into a single eye of each normal cynomolgus monkey. Then, intraocular pressure was measured before drug administration.
2. Then, each test compound solution was administered. To the vehicle group, 20 µl of the vehicle solution was instilled . To the bunazosin group, 20 µl of Detantol ophthalmic solution was instilled. To the latanoprost group, 20 µl of Xalatan ophthalmic solution was instilled . To the (bunazosin + latanoprost) group, 20 µl of Detantol ophthalmic solution was instilled and 5 minutes later 20 µl of Xalatan ophthalmic solugion was instilled .
3. 2, 4 and 6 hours after the administration of these test compound solutions, one drop of 0.4% oxybuprocain hydrochloride eye drops was instilled for topical anesthesia, and the intraocular pressure was measured. The maximum reduction of intraocular pressure in each group was determined by the following formula.

(Maximum reduction of intraocular pressure) = (intraocular

pressure at the time when the intraocular pressure was decreased

mostly) - (intraocular pressure before drug administration)

(Results and Discussion)

The experimental results are shown in Table 1.

Table 1

|  | Maximum reduction of intraocular pressure (mmHg) |
| --- | --- |
| Vehicle group | 0.9 |
| Bunazosin group | 1.9 |
| Latanoprost group | 2.6 |
| Bunazosin + latanoprost group | 3.0 |

As shown in Table 1, the intraocular pressure in the group dosed with bunazosin and latanoprost was lowered more than those in the bunazosin group and the latanoprost group. Those described above show that the combination of bunazosin and prostaglandins can bring about a remarkable reduction of intraocular pressure.

Industrial Applicability

The administration of bunazosin and prostaglandins in combination enhanced the intraocular pressure-lowering effect of each drug. Thus, the pharmaceutical composition of the invention is useful as a therapeutic agent of glaucoma.

**Claims**

1. A therapeutic agent of glaucoma, comprising bunazosin or a salt thereof and a prostaglandin in combination.

2. A therapeutic agent of glaucoma, comprising bunazosin or a salt thereof and a prostaglandin in combination, where these drugs mutually supplement and/or enhance the actions thereof.

3. A therapeutic agent of glaucoma according to claim 1 or 2, where the prostaglandin is latanoprost.

4. A method for treating glaucoma, comprising administration to a patient with a therapeutically effective amount of bunazosin or a salt thereof and a prostaglandin in combination .

5. A method for treating glaucoma according to claim 4, where the prostaglandin is latanoprost.

## INTERNATIONAL SEARCH REPORT

| International application No. |
| --- |
| PCT/JP03/00786 |

**A. CLASSIFICATION OF SUBJECT MATTER**
Int.Cl⁷ A61K31/5575, A61P27/06//(A61K31/5575, 31:551)

According to International Patent Classification (IPC) or to both national classification and IPC

**B. FIELDS SEARCHED**

Minimum documentation searched (classification system followed by classification symbols)
Int.Cl⁷ A61K31/5575, 31/551, A61P27/06

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN), BIOSIS(STN),
REGISTRY(STN), JICST(JOIS)

**C. DOCUMENTS CONSIDERED TO BE RELEVANT**

| Category* | Citation of document, with indication, where appropriate, of the relevant passages | Relevant to claim No. |
| --- | --- | --- |
| X | Ikuo AZUMA, 'Ryokunaisho Chiryoyaku no Shinpo', Journal of Japanese Ophthalmological Society, 1993 nen, Vol.97, No.2, pages 1353 to 1369, particularly, abstract<br>& Database Medline on STN, US National Library of Medicine (Bethesda, MD, USA), No.94120989 | 1-3 |
| X | WO 01/39805 A1 (Sankyo Co., Ltd.), 07 June, 2001 (07.06.01), Particularly, Abstract; claims<br>& EP 1234582 A1   & US 2003/040529 A1<br>& JP 2001-335511 A2   & AU 2001015575 A5<br>& BR 2000015980 A   & NO 2002002584 A | 1-3 |

| ☒ Further documents are listed in the continuation of Box C. | ☐ See patent family annex. |
| --- | --- |

\* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 21 April, 2003 (21.04.03) | 06 May, 2003 (06.05.03) |

| Name and mailing address of the ISA/<br>  Japanese Patent Office | Authorized officer |
| --- | --- |
| Facsimile No. | Telephone No. |

Form PCT/ISA/210 (second sheet) (July 1998)

6

| INTERNATIONAL SEARCH REPORT | International application No. |
|---|---|
| | PCT/JP03/00786 |

**C (Continuation). DOCUMENTS CONSIDERED TO BE RELEVANT**

| Category* | Citation of document, with indication, where appropriate, of the relevant passages | Relevant to claim No. |
|---|---|---|
| P,X | WO 02/45748 A1 (Sankyo Co., Ltd.), 13 June, 2002 (13.06.02), Particularly, Abstract; claims & JP 2002-234851 A & AU 2002018527 A5 | 1-3 |

Form PCT/ISA/210 (continuation of second sheet) (July 1998)

EP 1 479 387 A1

## INTERNATIONAL SEARCH REPORT

International application No.

PCT/JP03/00786

**Box I    Observations where certain claims were found unsearchable (Continuation of item 2 of first sheet)**

This international search report has not been established in respect of certain claims under Article 17(2)(a) for the following reasons:

1. ☒ Claims Nos.: 4, 5

   because they relate to subject matter not required to be searched by this Authority, namely:
   Claims 4, 5 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1998)